(19) Europäisches Patentamt
European Patent Office
Office européen des brevets

(11) Publication number: **0 041 934**
**B1**

(12) # EUROPEAN PATENT SPECIFICATION

(45) Date of publication of patent specification: **10.04.85**

(51) Int. Cl.⁴: **C 08 J 9/42,** A 61 L 15/01, A 61 L 15/03, A 01 N 59/12

(21) Application number: **81850104.1**

(22) Date of filing: **09.06.81**

(54) **Foamed plastic containing swellable polymer particles, and method for production thereof.**

(30) Priority: **11.06.80 SE 8004336**

(43) Date of publication of application:
**16.12.81 Bulletin 81/50**

(45) Publication of the grant of the patent:
**10.04.85 Bulletin 85/15**

(84) Designated Contracting States:
**AT BE CH DE FR GB IT LI LU NL SE**

(56) References cited:
**WO-A-79/00014**
**FR-A-2 147 839**
**FR-A-2 337 028**
**GB-A-1 001 912**
**GB-A-1 550 614**
**GB-A-1 570 485**

(73) Proprietor: **AB Ferrosan**
**Celsiusgatan 35**
**S-201 10 Malmö (SE)**

(72) Inventor: **Laszlo, Tomas Pablo Nicolas**
**Planetgatan 14**
**S-223 57 Lund (SE)**

(74) Representative: **Wiklund, Erik**
**AWAPATENT AB Box 5117**
**S-200 71 Malmö (SE)**

**EP 0 041 934 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a foamed plastic whose cavities consist of open cells or pores and which contains particles of water-insoluble hydrophilic polymer which is swellable to a gel in water. The invention also relates to a method of incorporating such polymer particles in a foamed plastic.

Particles or granules of water-insoluble, hydrophilic polymer which is swellable to a gel in water are previously known from, for example, Swedish patent specification 7301197-5. It is also known in the art to employ powder of such polymer particles for cleaning running sores, use being made of the capillary and absorption properties of the polymer particles for draining exudate from injured skin surfaces, particularly deep such injuries.

Apart from the absorbing properties, it is also known in the art to employ such polymer particles as carriers of active agents, such as disinfectants or pharmaceutically active agents. Hereby, a controlled progressive release of the active agent will be attained. An example of such employment is Austrian patent specification 334,548 which describes polymer particles of the above-mentioned type in which iodine has been incorporated. The thus obtained so-called gel iodophores combine the absorbing properties of the polymer particles with a storage of iodine, a protective effect being obtained which extenuates the chemically aggressive properties of the iodine at the same time as the storage life of the product in dry environment is extended.

These prior art wound powders suffer a number of drawbacks. Thus, there are difficulties in realizing suitable application forms for application to the skin and wounds. Direct application of the dry powder entails problems. in that removal from the treated surface is troublesome and time-consuming. Other conventional forms of application, such as ointments or the like, are not usable, since these considerably weaken or hinder the effect of the powder.

In the application process used hitherto, the powder is mixed with some suitable liquid, such as glycerol, the thus formed suspension being applied to the treatment surface, for example a running sore. This application process also involves disadvantages in that it requires instructed staff and regular change of the powder suspension because of the penetration of the particles into the wound and because of drying. These factors render an already expensive product even more costly and can in certain cases extend healing time as a result of the mechanical damage to the wound.

The object of the present invention is to obviate the problems and disadvantages inherent in prior art powders and to realize a powder-containing product which is easy to apply to and remove from a treatment surface, such as a running sore.

As was intimated by way of introduction, these problems and disadvantages are obviated in that the particles of water-insoluble, hydrophilic polym which is swellable to form a gel in water are incorporated in the cavities of a foamed plastic.

More precisely, the product according to the present invention is characterized in that the particles are substantially uniformly distributed in the cavities of the foamed plastic and anchored to the foamed plastic by means of a water-soluble binder, and the size of the particles is such that, in the unswelled state, they are smaller than the cavities of the foamed plastic and readily incorporable therein, whereas, in the swelled state, they substantially completely fill out the cavities in which they are incorporated.

Correspondingly, the method according to the present invention for incorporating such polymer particles in a foamed plastic whose cavities consist of open cells or pores is characterized in that the particles are suspended in a liquid medium which is substantially of the same density as the particles and in which a water-soluble binder is dissolved, that the foamed plastic is impregnated with the suspension and that the liquid medium is removed, the particles being retained in the foamed plastic by means of the binder, and the size of the particles being such that, in the unswelled state, they are readily incorporable in the cavities of the foamed plastic, whereas, in the swelled state, they substantially completely fill out the cavities in which they are incorporated.

As a result of the present invention, there is realized a dry product which may be applied to treatment surfaces such as wounds of varying geometric configuration and which, in a continuous manner, readily may be removed from the finally-treated surface. This entails a great step forward in relation to the prior art.

Furthermore, the foamed plastic product according to the invention has no hindering effect on the function of the polymer particles which may, instead, freely exercise their desired, inherent properties such as absorption or antibiotic or pharmaceutical activity in dependence upon the substances which have been encapsulated in the polymer particles. The foamed plastic product according to the invention permits, thus, the full application of the properties of the polymer particles at the same time as a steric hindrance is created for the polymer particles such that the system remains homogeneous when the polymer particles swell out to form a gel by utilizing the unifying network structure of the foamed plastic.

The invention also entails that the previous need for specially trained personnel and special instructions for use has been obviated. The wound treatment surface can be plugged and covered with pieces of the desired size of the foamed plastic product, which has a unifying effect on the incorporated polymer particles during the entire function process. Moreover, the foamed plastic product may readily be removed from the wound treatment surface without the

need for painful and thorough cleaning or rinsing of the wound. By incorporating the foamed plastic product according to the invention in suitable bandages, less regular and more simple changes of bandage are made possible, with the result that the patient is exposed to fewer painful manipulations and treatment is facilitated, thereby giving lower medical care costs.

The method according to the invention also involves a pluraltiy of advantages. In that the suspension with which the foamed plastic is impregnated, contains as a carrier a liquid of substantially the same density as the polymer particles, it is easy to suspend the polymer particles in the liquid and the polymer particles will remain suspended in this liquid. Since the polymer particles, because they are of substantially the same density as the liquid, remain suspended in the liquid and substantially lack any inclination to form a sediment, the polymer particles will effortlessly accompany the liquid on impregnation of the foamed plastic, whereby the polymer particles are homogeneously distributed in the cavities of the foamed plastic.

In order that it be possible for the polymer particles to be incorporated in the foamed plastic, the particles are, naturally, of smaller dimensions than the open cells or pores of the foamed plastic. This entails the risk that the polymer particles separate from the foamed plastic if they are not in some way retained therein. To this end, the impregnating agent includes a binder which is dissolved in the impregnating agent. When, after impregnation, the carrier liquid is removed, the binder acts to retain the polymer particles in the cavities of the foamed plastic. The sole object of the binder is to exercise this retaining function in the preparation and storage of the foamed plastic product. When the foamed plastic product is used, the polymer particles swell up such that they substantially fill out the cavities in which they have been incorporated, thereby creating a steric hindrance which prevents the polymer particles from separating from the foamed plastic. On use of the foamed plastic product, the binder is, thus, not necessary since its function ceases at the same time as it is dissolved in the exudate and is replaced by the swelling ability of the polymer particles. This is realized by using a water-soluble binder which, thus, is dissolved on use and departs from the foamed plastic product. Consequently, the binder in the present invention should be soluble both in the liquid carrier and in water.

One disadvantage inherent in the incorporation of the binder may be that it has a rigidifying effect on the foamed plastic and reduces the yieldability and flexibility of the plastic. For this reason, it is appropriate to incorporate into the liquid carrier a substance acting as a plasticizer and surfactant, such as a so-called poloxamer, that is to say a polyoxyethylene polyoxypropylene block copolymer. This counteracts possible rigidification and keeps the binder and foamed plastic yieldable and flexible so that the foamed plastic may easily be adapted to the configuration of different treatment surfaces.

A more detailed description will be given below of the components and methods utilized in the invention and further advantages inherent in the present invention will thereby be disclosed.

The particles of water-insoluble, hydrophilic polymer which is swellable to a gel in water and is used in the present invention, consist of per se known products, such as those which, for example, are described in the above-mentioned Swedish and Austrian patent specifications. In view hereof, no detailed description of these prior art polymer particles will be necessary, but for the sake of completeness, a brief outline of these prior art products will be given.

Swedish patent specification 7301197-5 describes the use of liquid-absorbing material for cleaning running skin, wound and mucous membrane surfaces. The materials involved here are substantially particles of dextran or carboxy methyl dextran which have been cross-linked by means of epichlorohydrin. The hydrophilic polymer particles are swellable to a limited degree to form a gel in water and the water absorption is given as about 0.5 to 30 g/g polymer, preferably about 2—5 g/g polymer. The mean particle size of the particles is given as about 10 to 1000 µm. Polymer particles of the above-disclosed type are marketed by Pharmacia AB, Sweden, under the trademark DEBRISAN®.

Austrian patent specification 334,548 describes a disinfectant and a method for its preparation, the disinfectant containing iodine which is complexed to a hydrophilic organic carrier which is swellable in water under the formation of gel. The carrier may consist of a plurality of materials such as, for example, those disclosed in the above-mentioned Swedish patent specification and comprises, for example, cross-linked dextran, dextrin, starch and starch derivatives, sorbitol, saccharose and polyvinyl alcohol. The water absorption capacity or swellability of the carrier is given as about 2 to 100 ml/g and the content of complexed iodine at up to about 5 to 10% iodine. A particle size of about 0.01 to 1 mm of the carrier is also mentioned.

Recently, extremely high-absorbing, hydrophilic polymers with an absorption capacity of about 1000—1200 ml/g have become known, and these polymers are also included for use as swellable polymer particles in the present invention. These high-absorbing polymers which are also known under the designations "Superslurper", "Stasorb" and "H-Span", consist of graft copolymers of starch and starch derivatives, respectively, and vinyl monomers, such as acrylonitrile, methyl methacrylate and acryl amide. By incorporating, in the present invention, such polymers in a foamed plastic, a unifying unit is created which, in a manner of speaking, reinforces the high-absorbing polymer. For more detailed information concerning these high-absorbing polymers, reference is made to an article by Rakesh Mehrotra and Bengt Rånby

entitled "Graft Copolymerisation onto Starch", Journal of Applied Polymer Science, Vol. 21, 1977, pp. 1647—1654.

The above-described polymer particles should only be seen as examples of particles of hydrophilic, water-insoluble polymers which are swellable in water to form a gel and which may be used in the invention, and it will be understood that other polymer particles which satisfy the requirements placed on water insolubility, hydrophility and swellability in water to form a gel may also be used in the invention and thus fall in the spirit and scope of the invention as claimed.

In purely general terms, it is possible according to the present invention, to use, as such polymer particles, any type of water-insoluble hydrophilic polymer which is swellable in water to form a gel. The polymer is in the form of irregular or preferably spherical particles. As specific examples of such polymers mention may be made of starch, dextran, dextrin, cellulose, saccharose, maltose, lactose, sorbitol or similar organic polyhydroxyl group-containing materials which have been cross-linked with the assistance of bifunctional, organic cross-linking agents such as epichlorohydrin. The swellability in water of these polymers amounts in the invention to as much as about 1200 ml/g, but normally varies between about 2 and 100 ml/g. As was mentioned earlier, the swellable polymer particles in the invention may include biologically active substances, such as disinfectants or pharmaceutically active substances which are released and activated when the polymer particles are brought into contact with water or biological liquids. The particle size of the polymer particles in the invention amounts to about 10 to 1000 µm, suitably about 20 to 500 µm and preferably about 20 to 100 µm. As regards the particle size, it may be said in general that the size is dependent upon the size of the cavities of the foamed plastic, and the particle size of the polymer particles in the unswelled state should be less than the diameter of the cavities of the foamed plastic, whereas the particle size in the swelled state should be such that the polymer particles substantially completely fill out the cavities in the foamed plastic in which they are located. The first condition for particle size has been set out in order that the polymer particles be easily incorporable into the foamed plastic, while the second condition is based on the concept that the swellable polymer particles shall be positively retained by the network structure of the foamed plastic by steric hindrance.

In order to define more closely the particle size of the polymer particles and the relationship between this and the cavity size in the foamed plastic, the following theoretical reasoning may be presented:

$$V_{gs}=S\times V_{gt},$$

wherein

$V_{gs}$ is the volume of the polymer particles in the swelled state,

$V_{gt}$ is the volume of the polymer particles in the dry state, and

S is the swelling factor.

For the foamed plastic, the following equation is correspondingly valid.

$$V_{pf}=F\times V_{pt},$$

wherein

$V_{pf}$ is the cavity volume of the foamed plastic in the moist state,

$V_{pt}$ is the cavity volume of the foamed plastic in the dry state, and

F is the swelling factor of the foamed plastic.

On incorporation of polymer particles in the cavities of the foamed plastic, the following equation is valid

$$V_{gs}\geq V_{pf},$$

that is to say

$$S\times V_{gt}\geq F\times V_{pt}.$$

If the foamed plastic does not change its volume in the moist state, F=1. If, at the same time, the polymer particles swell in water to 5 times their original volume, that is to say S=5, the following relationship will apply:

$$V_{gt}\geq V_{pt}/5$$

which entails that the volume of the dry polymer particles should be at least 1/5 of the cavity volume of the foamed plastic in the dry state.

In addition, there is the desired filling degree, that is to say the percentage of the cavities of the foamed plastic in the form of pores or open cells which are filled with polymer particles. In general, it can be said that at least about 1% and preferably at least 10% of the cavities of the foamed plastic should be provided with swellable polymer particles in order that the object of the invention be attained. A preferred filling degree is about 40—50%, at which the capillarity of the foamed plastic is retained. In certain cases when it is desired fully to utilize the capillarity of the swelled polymer particles, a filling degree as high as about 90—100% may be desirable.

It may be added that the above reasoning is applicable both when the cavities of the foamed plastic contain and are filled out by individual polymer particles, that is to say one single polymer particle has the capacity, on swelling, to fill out the cavity in which it has been incorporated, and when the cavities of the foamed plastic contain and are filled out by polymer particles which consist of aggregate bodies of smaller primary particles. In the latter case, the above reasoning applies to the aggregate body of primary particles.

The foamed plastic which is selected for use in the present invention may be of known type. Thus, the invention is not restricted to foamed plastics of any certain type or any certain

material, apart from the condition that the cavities of the foamed plastic must consist of open cells or pores. This is an obvious condition in order that the foamed plastic be impregnated with polymer particles. The object of the foamed plastic is to provide a suitable network structure as carrier for the polymer particles, and usable foamed plastics are, here, non-reticulated foamed plastics having good permeability, or reticulated foamed plastics, depending upon the desired thickness of the foamed plastic (and thereby the permeability to liquid and particles). By reticulated foamed plastic is intended a foamed plastic of three-dimensional structure which defines numerous individual cells in which the majority or all cell wall membranes which define the cells are removed such that only the skeleton of the three-dimensional structure remains. The term pores is taken to mean pervading, open channels in the foamed plastic.

Normally, use is made of a foamed plastic which does not (or at least does not substantially) swell on contact with aqueous solutions, but the invention does not exclude the use of foamed plastics having special properties, such as absorbing and/or swellable foamed plastics for special purposes. Hydrophilic foamed plastics are also covered by the present invention. In particular in those cases when the foamed plastic product according to the invention is intended for biological use, such as the cleaning of running sores, it is suitable to utilize specially cleansed foamed plastic, that is to say foamed plastic of standard quality which, through chemical purification, has been freed from soluble components which may have a contaminating effect in biological use.

The foamed plastics according to the invention have a porosity of about 5—200 ppi (pores per inch), which corresponds to a pore size of about 0.02—5 mm in diameter. In view of that which has been mentioned above the skilled reader of this specification will realize that the pore size of the foamed plastic must be adapted to the size and swelling capacity of the polymer particles. Furthermore, a certain attention should be paid to the desired thickness of the foamed plastic, and a slight pore size should not be selected when the foamed plastic is very thick.

A particularly preferred type of the foamed plastic in the present invention is polyester or polyether based foamed polyurethane plastic. In particular, polyester based foamed polyurethane plastic is here to be preferred, since this makes for a homogeneous and well-defined pore size which is a desired property in the invention in view of the incorporation of the polymer particles.

It is also possible, and in certain contexts advisable, to laminate the foamed plastic on one side with a layer of another material, such as a non-woven layer.

As has been mentioned above, the foamed plastic product according to the present invention includes a water-soluble binder whose object it is to anchor the polymer particles to the foamed plastic on preparation and storage of the foamed plastic product. A critical condition for the binder in the invention is that it be soluble both in the liquid impregnating agent and in the aqueous medium in order that the binder may easily be supplied together with the impregnating agent on preparation of the foamed plastic product, and also removed on use of the foamed plastic product.

Examples of preferred binders in the invention are polyvinyl pyrrolidone (PVP) and copolymers thereof, such as vinyl pyrrolidone-vinyl acetate copolymers (VP/VA, $M_w$=40,000—90,000), polyvinyl alcohols (PVA) and poly - 2 - methyl - 5 - vinyl pyridine (PMVP). PVP has in general a molecular weight, $M_w$, of from about 2500∓1000 to 700,000, PVP of a lower molecular weight of up to about 40,000 being preferred for biological purposes.

As was mentioned previously, it is also advisable in the present invention to incorporate into the impregnating agent a plasticizing surface active agent. Like the binder, this substance should be soluble both in the impregnating agent and in water. Similarly, both the binder and the plasticizing and surface active agent should be pharmaceutically acceptable when the foamed plastic product is intended for biological use, such as the cleaning of wounds.

Particularly preferred plasticizing and surface active agents in the invention consist of so-called poloxamers which are polyoxyethylene-polyoxypropylene block copolymers of the general formula

$$HO(CH_2CH_2O)_a(\underset{\displaystyle CH_3}{\underset{|}{CH}}\,CH_2O)_b(CH_2CH_2O)_cH$$

the compositions in which b constitutes 5—25% and a+c constitutes 75—95% of the total weight of the block copolymer and $M_w$ is 5000—15500 being particularly preferred. One particular example of such a poloxamer is PLURONIC® F-87 also designated poloxamer 237, whose hydrophobic portion (polyoxypropylene portion) has a molecular weight of about 2250, whereas the hydrophilic portion constitutes about 70% of the total molecular weight.

The contents employed in the invention, of binder and plasticizing and surface active agent are selected such that the best results as regards binding and plasticizing properties are realized. Particularly good results have been obtained in an amount ratio of polymer particles to binder+plasticizer of between 4:1 and 2:1. It is moreover preferred that the amount ratio of binder to plasticizer lies between 1:4 and 250:4.

As was mentioned earlier, a liquid impregnating agent or carrier is utilized in the incorporation of the polymer particles in the foamed plastic. The conditions which apply to this medium are that it should be of substantially the same density as the polymer particles, it should not have a dissolving or swelling effect on the polymer particles, it should not have a dissolving

effect on the foamed plastic, it should be a solvent to the binder and any other additives, and it should, furthermore, be easy to remove from the foamed plastic, for example, in that it is volatile. The liquid medium need not be made up of a solitary liquid but may be composed of a mixture of two or more liquids.

With the knowledge of the above conditions, a person skilled in this art can easily establish by tests the composition of a suitable liquid medium. As suitable impregnating agents in the invention, use has been made of mixtures of ethanol/chloroform and ethanol/carbon tetrachloride.

For the impregnation of the foamed plastic, a binder and suitably also a plasticizing and surface active agent are added to the impregnating agent and are dissolved therein. The content of binder in the impregnating agent is suitably selected to be about 1—20 weight %, preferably about 2—10 weight % and most preferably about 3—5 weight %. The possible additive of plasticizing and surface active agent is selected according to earlier disclosed relationships. Moreover, to the impregnating agent are added polymer particles to a content of about 1—80 weight %.

This content depends upon the desired degree of filling, but it is preferably about 10—50 weight % and most preferably about 20—40 weight %. Once all of the additions have been made to the impregnating agent, it is ready to be used for impregnating the foamed plastic.

The actual method for impregnating the foamed plastic is not vital, that is to say the invention is not bound to any particular method for impregnating the foamed plastic; all methods which realize a homogeneous distribution of the polymer particles in the foamed plastic are applicable to the present invention. As examples of usable methods, mention may be made of impregnation according to the so-called Foulard technique, application by spraying or other methods which are used in, for example, the textile industry. The Foulard technique implies, in brief, that the foamed plastic is immersed in a bath with the impregnating agent by guiding by means of, for example, rollers, the impregnating agent penetrating into the foamed plastic. Thereafter, the foamed plastic is passed up and out of the bath and through the nip of two rollers for squeezing out excess impregnating agent. Once the incorporated amount of impregnating agent has been regulated in this manner, the foamed plastic is passed into a drying apparatus, such as a chamber with forced circulation of air at a temperature which varies from room temperature up to about 100°C. When the foamed plastic has passed the drying apparatus, it is dry and ready for delivery.

Spraying implies that the foamed plastic is passed beneath a spraying nozzle which sprays impregnating agent onto the foamed plastic, whereafter the foamed plastic, as in the Foulard technique, is led through the nip of two rollers for squeezing out excess impregnating agent, and then through a drying apparatus.

As will be apparent from the above description, the product according to the invention is primarily intended for clinical use, such as the treatment of running sores, in complaints involving incontinence and the like in which its absorption is of particular value. The product according to the invention is not, however, restricted to clinical use, but may be used also in technical applications, such as disinfection of polluted water in, for example, swimming pools, cooling towers and the like. In such forms of application, polymer particles which include disinfectants are used, such as the previously described gel iodophores.

The main features in the aspects of the present invention having been presented above, the invention will now be described by means of some illustrative, but not restrictive, examples to further facilitate understanding of the invention.

Example 1

3 g of polyvinylpyrrolidon (Kollidon 25, $M_w$ approx. 30,000) and 1 g of poloxamer 187 (Pluronic® F 64, $M_w$ of the hydrophobic portion 1750; the hydrophilic portion constitutes 70% of the total $M_w$) were dissolved in a liquid impregnating agent consisting of a mixture of 77 ml carbon tetrachloride and 22 ml ethanol (95%). Thereafter, 33 ml of polymer particles of cross-linked dextran epichlorohydrin (Sephadex® G 25 Coarse, Pharmacia) were added to the impregnating agent. The mean particle size of the particles was 100 to 300 μm, and 1 g of particles absorbed up to 4 ml of water.

A 2 mm thick and 100 mm broad strip of foamed plastic of reticulated, polyester-based polyurethane of a pore size of 0.2—0.3 mm was impregnated with the thus prepared impregnating agent. The impregnation took place according to the Foulard technique with the help of an impregnation bath, rolling and drying. By regulating the roller pressure, the amount of incorporated polymer particles was adapted to from 0.4—0.5 ml of dry particles/$cm^2$ of foamed plastic.

A dry foamed plastic product with homogeneously distributed polymer particles was thus obtained, the product flexibly adapting itself to a wound surface on application thereto and being easy to remove once the foamed plastic product had fulfilled its function and sucked up exudate from the wound.

Example 2

3.5 g of polyvinylpyrrolidone (Kollidon 12 PF from BASF of an $M_w$ of 2500±1000) and 0.2 g poloxamer 237 (Pluronic® F-87) were dissolved in a mixture consisting of 19 ml ethanol (99.5%) and 78 ml carbon tetrachloride.

In the above solution, 25 g of polymer particles of the type described in Example 9 in Austrian Patent 334,584 (iodophore gel) were suspended, the particles consisting of iodine-containing dextran gel (2.39% iodine).

The thus obtained suspension was used as an impregnating agent for impregnating a 0.5 cm

thick and 10 cm wide strip of non-reticulated polyester-based foamed polyurethane plastic having hydrophilic properties, the foamed plastic having a pore size of 0.30—0.34 mm.

After impregnation by means of the Foulard technique and drying, a foamed plastic product was obtained which contained 0.21 g of homogeneously distributed polymer particles per $cm^3$ of foamed plastic.

Example 3

Example 1 was repeated but with the difference that the liquid impregnating agent was a solvent mixture consisting of 84 ml of chloroform and 15 ml of ethanol (95%) instead of carbon tetrachloride/ethanol.

After impregnation using the Foulard technique and drying, a foamed plastic product was obtained having the same excellent properties as that in Example 1.

Example 4

Example 1 was repeated, but with the difference that the foamed plastic strip had a thickness of 20 mm instead of 2 mm.

After impregnation, rolling and drying in the same manner as in Example 1, a foamed plastic product was obtained which, similarly, contained homogeneously distributed polymer particles despite the greater thickness.

**Claims**

1. Foamed plastic whose cavities consist of open cells or pores and which contains particles of water-insoluble, hydrophilic polymer which is swellable to form a gel in water, characterized in that the particles are substantially uniformly distributed in the cavities of the foamed plastic and anchored to the foamed plastic by a water-soluble binder, the size of the particles being such that, in the unswelled state, they are smaller than the cavities of the foamed plastic and readily incorporable therein, whereas, in the swelled state, they substantially completely fill out the cavities in which they are incorporated.

2. The foamed plastic as claimed in claim 1, characterized in that at least 10% of the cavities of the foamed plastic are provided with particles of water-insoluble, hydrophilic, swellable polymer.

3. The foamed plastic as claimed in claim 1 or 2, characterized in that the foamed plastic consists of polyurethane.

4. The foamed plastic as claimed in any one of claims 1—3, characterized in that the particles of the water-insoluble, hydrophilic, swellable polymer are selected from among polymers based on starch, dextran, dextrin, cellulose, saccharose, maltose, lactose or sorbitol.

5. The foamed plastic as claimed in claim 4, characterized in that the particles consist of cross-linked dextran.

6. The foamed plastic as claimed in any one of the preceding claims, characterized in that the particles include disinfecting or pharmaceutically active agents.

7. The foamed plastic as claimed in any one of the preceding claims, characterized in that the water-soluble binder is selected from among polymers or copolymers based on vinyl alcohol or vinyl pyrrolidone.

8. The foamed plastic as claimed in any one of the preceding claims, characterized in that a poloxamer consisting of a block copolymer of polyoxy-ethylene and polyoxypropylene has been added to the binder.

9. A method of incorporating particles of water-insoluble, hydrophilic polymer which is swellable to form a gel in water, in a foamed plastic whose cavities consist of open cells or pores, characterized in that the particles are suspended in a liquid medium in which the particles do not dissolve or swell and which is of substantially the same density as the particles and wherein is dissolved a water-soluble binder, that the foamed plastic is impregnated with the suspension, and that the liquid medium is removed, the particles being retained in the foamed plastic by means of the binder, and the size of the particles being such that, in the unswelled state, they are readily incorporable in the cavities of the foamed plastic, whereas, in the swelled state, they substantially completely fill out the cavities in which they are incorporated.

10. The method as claimed in claim 9, characterized in that the foamed plastic is immersed in the suspension and, after withdrawal therefrom, is passed between a pair of rollers for squeezing out excess suspension.

11. The method as claimed in claim 9 or 10, characterized in that the liquid medium is selected from among alcohols and chlorinated hydrocarbons, and mixtures thereof.

12. The method according to claim 11, characterized in that the liquid medium is selected from among ethanol, chloroform, carbon tetrachloride, or mixtures thereof.

**Patentansprüche**

1. Schaumkunststoff, dessen Hohlräume aus offenen Zellen oder Poren bestehen und der Teilchen eines wasserunlöslichen, hydrophilen Polymers enthält, welches in Wasser zu einem Gel quellbar ist, dadurch gekennzeichnet, dass die Teilchen hauptsächlich gleichförmig in den Hohlräumen des Schaumkunststoffs verteilt und mittels eines wasserlöslichen Bindemittels am Schaumkunststoff verankert sind, wobei die Grösse der Teilchen eine solche ist, dass die im ungequellten Zustand kleiner sind als die Hohlräume des Schaumkunststoffs und leicht darin einführbar sind, während sie im gequellten Zustand die Hohlräume, in denen sie eingelagert sind, im wesentlichen völlig ausfüllen.

2. Schaumkunststoff nach Anspruch 1, dadurch gekennzeichnet, dass zumindest 10% der Hohlräume des Schaumkunststoffs mit Teilchen

eines wasserunlöslichen, hydrophilen, quellfähigen Polymers versehen sind.

3. Schaumkunststoff nach Anspruch 1 oder 2, dadurch gekennzeichnet, dass der Schaumkunststoff Polyurethan ist.

4. Schaumkunststoff nach einem der Ansprüche 1—3, dadurch gekennzeichnet, dass die Teilchen des wasserunlöslichen, hydrophilen, quellfähigen Polymers unter auf Stärke, Dextran, Dextrin, Cellulose, Saccharose, Maltose, Lactose oder Sorbitol basierten Polymeren gewählt sind.

5. Schaumkunststoff nach Anspruch 4, dadurch gekennzeichnet, dass die Teilchen aus vernetztem Dextran bestehen.

6. Schaumkunststoff nach einem der vorhergehenden Ansprüche, dadurch gekennzeichnet, dass die Teilchen Desinfektionsmittel oder pharmazeutisch aktive Stoffe enthalten.

7. Schaumkunststoff nach einem der vorhergehenden Ansprüche, dadurch gekennzeichnet, dass das wasserlösliche Bindemittel unter auf Vinylalkohol oder Vinylpyrrolidon basierten Polymeren oder Copolymeren gewählt ist.

8. Schaumkunststoff nach einem der vorhergehenden Ansprüche, dadurch gekennzeichnet, dass dem Bindemittel ein Poloxamer hinzugesetzt ist, das aus einem Blockcopolymer von Polyoxyäthylen und Polyoxypropylen besteht.

9. Verfahren zur Einlagerung von Teilchen eines wasserunlöslichen, hydrophilen Polymers, das in Wasser zu einem Gel quellfähig ist, in einen Schaumkunststoff, dessen Hohlräume aus offenen Zellen oder Poren bestehen, dadurch gekennzeichnet, dass die Teilchen in einem flüssigen, die Teilchen nicht auflösenden oder nicht zum Quellen bringenden Mittel suspendiert sind, das im wesentlichen dieselbe Dichte wie die Teilchen hat und in dem ein wasserlösliches Bindemittel aufgelöst ist, dass der Schaumkunststoff mit der Suspension imprägniert wird, und dass das flüssige Mittel entfernt wird, wobei die Teilchen mit Hilfe des Bindemittels im Schaumkunststoff zurückgehalten werden, und wobei die Grösse der Teilchen eine solche ist, dass sie im ungequellten Zustand leicht in die Hohlräume des Schaumkunststoffs eingelagert werden können, während sie im gequellten Zustand die Hohlräume, in denen sie eingelagert sind, im wesentlichen völlig ausfüllen.

10. Verfahren nach Anspruch 9, dadurch gekennzeichnet, dass der Schaumkunststoff in die Suspension eingetaucht und nach Herausnahme aus der Suspension zwischen zwei Walzen geleitet wird, um überschüssige Suspension auszuquetschen.

11. Verfahren nach Anspruch 9 oder 10, dadurch gekennzeichnet, dass das flüssige Mittel unter Alkoholen und chlorinierten Kohlenwasserstoffen sowie Gemischen davon gewählt wird.

12. Verfahren nach Anspruch 11, dadurch gekennzeichnet, dass das flüssige Mittel unter Äthanol, Chloroform, Kohlenstofftetrachlorid oder Gemischen davon gewählt wird.

**Revendications**

1. Mousse plastique dont les cavités sont constituées de cellules ou pores ouverts et qui contient des particules d'un polymère hydrophile insoluble dans l'eau qui est gonflable dans l'eau pour former un gel, caractérisée en ce que les particules sont distribuées de façon pratiquement uniforme dans les cavités de la mousse plastique et ancrées à la mousse plastique par un liant soluble dans l'eau, la taille des particules étant telle qu'à l'état non gonflé elles soient plus petites que les cavités de la mousse plastique et y soient facilement incorporables, tandis qu'à l'état gonflé elles remplissent pratiquement totalement les cavités dans lesquelles elles sont incorporées.

2. La mousse plastique comme revendiqué dans la revendication 1, caractérisée en ce qu'au moins 10% des cavités de la mousse plastique contiennent des particules de polymère gonflable, hydrophile, insoluble dans l'eau.

3. La mousse plastique selon la revendication 1 ou 2, caractérisée en ce que la mousse plastique est constituée de polyuréthanne.

4. La mousse plastique comme revendiqué dans l'une quelconque des revendications 1 à 3, caractérisée en ce que les particules du polymère gonflable, hydrophile, insoluble dans l'eau sont choisies parmi les polymères à base d'amidon, de dextran, de dextrine, de cellulose, de saccharose, de maltose, de lactose ou de sorbitol.

5. La mousse plastique comme revendiqué dans la revendication 4, caractérisée en ce que les particules sont constituées de dextran réticulé.

6. La mousse plastique comme revendiqué dans l'une quelconque des revendications précédentes, caractérisée en ce que les particules comprennent des agents désinfectants ou à activité pharmaceutique.

7. La mousse plastique comme revendiqué dans l'une quelconque des revendications précédentes, caractérisée en ce que le liant soluble dans l'eau est choisi parmi les polymères ou copolymères à base d'alcool vinylique ou de vinylpyrrolidone.

8. La mousse plastique comme revendiqué dans l'une quelconque des revendications précédentes, caractérisée en ce qu'un poloxamère constitué d'un copolymère séquencé de polyoxyéthylène et de polyoxypropylène a été ajouté au liant.

9. Un procédé d'incorporation de particules d'un polymère hydrophile insoluble dans l'eau qui est gonflable dans l'eau pour former un gel, dans une mousse plastique dont les cavités sont constituées de cellules ou pores ouverts, caractérisé en ce que les particules sont mises en suspension dans un milieu liquide dans lequel les particules ne se dissolvent pas ni ne gonflent et qui a essentiellement la même densité que les particules, et dans lequel un liant soluble dans l'eau est dissous, la mousse plastique est imprégnée de la suspension et le milieu liquide

est éliminé, les particules étant retenues dans la mousse plastique par le liant, et la taille des particules étant telle qu'à l'état non gonflé elles soient facilement incorporables dans les cavités de la mousse plastique, tandis qu'à l'état gonflé elles remplissent essentiellement complètement les cavités dans lesquelles elles sont incorporées.

10. Le procédé de la revendication 9, caractérisé en ce que la mousse plastique est plongée dans la suspension et, après en avoir été retirée, est conduite entre une paire de rouleaux pour chasser l'excès de suspension.

11. Le procédé comme revendiqué dans la revendication 9 ou 10, caractérisé en ce que le milieu liquide est choisi parmi les alcools, les hydrocarbures chlorés et leurs mélanges.

12. Le procédé selon la revendication 11, caractérisé en ce que le milieu liquide est choisi parmi l'éthanol, le chloroforme, le tétrachlorure de carbone ou leurs mélanges.